# EUROPEAN PATENT APPLICATION

(11) **EP 4 378 423 A1**
(43) Date of publication of application: **05.06.2024**
(21) Application number: 23197704.2
(22) Date of filing: 15.09.2023
(51) Int. Cl.: A61F 2/24

(54) **VALVE PROSTHESIS WITH DURABLE PROSTHETIC VALVE**

(30) Priority: 04.10.2022 US 202263413067 P; 08.09.2023 US 202318464036
(71) Applicant: Medtronic Vascular Inc., Santa Rosa, CA 95403 (US)
(72) Inventor: Olney, Karl L., Santa Ana, 92705 (US); Kibria, Alkindi, Santa Ana, 92705 (US); Cheshko, Tasha, Santa Ana, 92705 (US); Dorman, Kyle J., Santa Ana, 92705 (US)
(74) Representative: Zimmermann & Partner Patentanwälte mbB

(57) **Abstract**

The techniques of this disclosure generally relate to a valve prosthesis having a one piece molded prosthetic valve and a reinforcement member attached to the prosthetic valve directly adjacent a cylindrical inflow end of the prosthetic valve. The reinforcement member is additional material outside the leaflet belly region that gets attached directly to a stent structure to provide reinforcement and durability of the prosthetic valve.

## Description

### CROSS-REFERENCE TO RELATED APPLICATIONS

This application claims the benefit of U.S. Provisional Application No. 63/413,067, filed October 4, 2022, the contents of which are incorporated by reference herein in their entirety.

### FIELD

The present technology is generally related to transcatheter heart valves.

### BACKGROUND

A human heart includes four heart valves that determine the pathway of blood flow through the heart: the mitral valve, the tricuspid valve, the aortic valve, and the pulmonary valve. The mitral and tricuspid valves are atrioventricular valves, which are between the atria and the ventricles, while the aortic and pulmonary valves are semilunar valves, which are in the arteries leaving the heart. Ideally, native leaflets of a heart valve move apart from each other when the valve is in an open position, and meet or "coapt" when the valve is in a closed position. Problems that may develop with valves include stenosis in which a valve does not open properly, and/or insufficiency or regurgitation in which a valve does not close properly. Stenosis and insufficiency may occur concomitantly in the same valve. The effects of valvular dysfunction vary, with regurgitation or backflow typically having relatively severe physiological consequences to the patient.

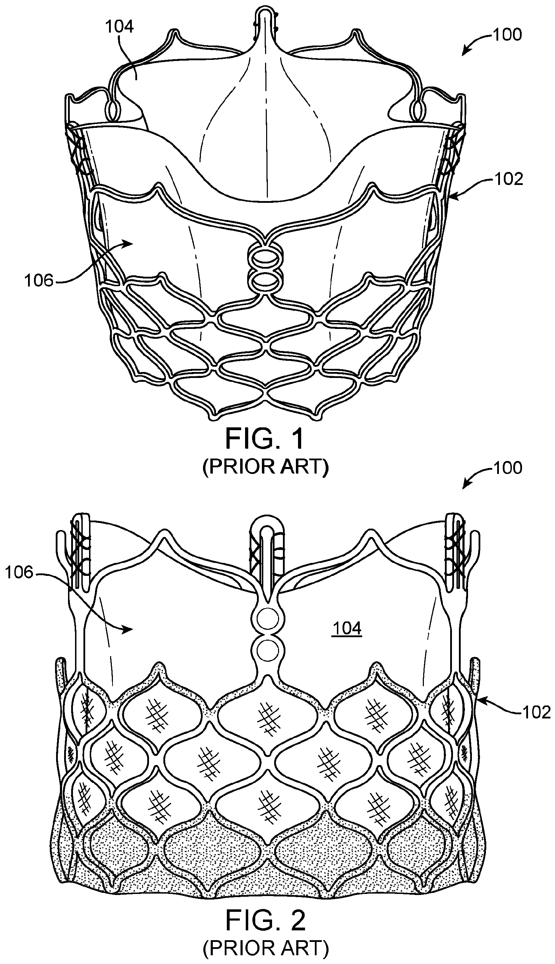

Recently, flexible prosthetic valves supported by stent structures that can be delivered percutaneously using a catheter-based delivery system have been developed for heart and venous valve replacement. FIG. 1 is a perspective view of a valve prosthesis 100 in accordance with the prior art. FIG. 2 is a side view of valve prosthesis 100 of FIG. 1 in accordance with the prior art. Referring to FIGS. 1 and 2, valve prosthesis 100 may include a balloon-expandable stent structure 102 with valve leaflets 104 attached to the interior of stent structure 102. Valve leaflets 104 may be parts of a 3D single piece prosthetic valve 106.

Valve prosthesis 100 can be reduced in diameter, by crimping onto a balloon catheter, and advanced through the venous or arterial vasculature. Once valve prosthesis 100 is positioned at the treatment site, for instance within an incompetent native valve, stent structure 102 may be expanded to hold valve prosthesis 100 firmly in place.

When designing a valve prosthesis such as valve prosthesis 100, valve-frame integration and frame mechanical performance often have competing needs or requirements. For example, when attaching the valve to the frame during valve-frame integration, the valve itself needs to be reinforced to the frame at certain locations without hindering mechanical performance of the frame. Embodiments hereof relate to an improved balloon-expandable transcatheter valve prosthesis configured to minimize tradeoffs between the above-described competing needs.

### SUMMARY

The techniques of this disclosure generally relate to a valve prosthesis having a one piece molded prosthetic valve and a reinforcement member attached to the prosthetic valve directly adjacent a cylindrical inflow end of the prosthetic valve. The reinforcement member is additional material outside the leaflet belly region that gets attached directly to a stent structure to provide reinforcement and durability of the prosthetic valve.

In one aspect, the present disclosure provides a valve prosthesis having a one piece molded prosthetic valve having a valve leaflet including a cusp and a valve inflow cylinder proximal of the valve leaflet. The valve prosthesis further includes a reinforcement member attached to the valve inflow cylinder adjacent the cusp. The reinforcement member is located between the cusp and a stent structure preventing the valve leaflet from hitting or otherwise contacting the stent structure. At the same time, the reinforcement member is located at a margin of attachment of the prosthetic valve to the stent structure to provide more strength as compared to the prosthetic valve alone.

In another aspect, the present disclosure provides a valve prosthesis having a one piece molded prosthetic valve, a stent structure, an inner skirt, and tacking stitching. The prosthetic valve has valve leaflets and a valve inflow cylinder proximal of the valve leaflets. The inner skirt is attached to the stent structure. The tacking stitching tacks the valve inflow cylinder to the inner skirt. The tacking stitching provides additional tacking to avoid undesirable bulging of prosthetic valve, e.g., bulging inward and away from the stent structure.

Further disclosed herein is a valve prosthesis having a one piece molded prosthetic valve and a reinforcement member attached to the prosthetic valve directly adjacent a cylindrical inflow end of the prosthetic valve, wherein the reinforcement member is additional material outside the leaflet belly region that gets attached directly to a stent structure to provide reinforcement and durability of the prosthetic valve.

The details of one or more aspects of the disclosure are set forth in the accompanying drawings and the description below. Other features, objects, and advantages of the techniques described in this disclosure will be apparent from the description and drawings, and from the claims.

### BRIEF DESCRIPTION OF DRAWINGS

FIG. 1 is a perspective view of a valve prosthesis in accordance with the prior art.
FIG. 2 is a side view of the valve prosthesis of FIG. 1 in accordance with the prior art.
FIG. 3 is a perspective view of a valve prosthesis in an expanded configuration in accordance with one embodiment.
FIG. 4 is an outflow end view of a prosthetic valve of the valve prosthesis of FIG. 3 in accordance with one embodiment.
FIG. 5 is a perspective view of the prosthetic valve including a reinforcement member of the valve prosthesis of FIG. 3 in accordance with one embodiment.
FIG. 6 is a side view of the valve prosthesis of FIG. 3 in accordance with one embodiment.
FIG. 7 is an enlarged cross-sectional view of a region VII of the prosthetic valve including the reinforcement member of FIG. 5 including a stent structure of the valve prosthesis of FIG. 3 in accordance with one embodiment.
FIG. 8 is a perspective view of the prosthetic valve including reinforcement members of the valve prosthesis of FIG. 3 in accordance with another embodiment.
FIG. 9 is an enlarged plan view of a region IX of the prosthetic valve and a reinforcement member of FIG. 8 in accordance with one embodiment.
FIG. 10 is an enlarged cross-sectional view of the region IX of the prosthetic valve including the reinforcement member of FIG. 8 including the stent structure of the valve prosthesis of FIG. 3 in accordance with one embodiment.
FIG. 11 is a laid flat plan view of a tissue coupon illustrating the prosthetic valve including the reinforcement members of FIGS. 8-10 in accordance with one embodiment.
FIG. 12 is a perspective view of the prosthetic valve including reinforcement stitching of the valve prosthesis of FIG. 3 in accordance with another embodiment.
FIG. 13 illustrates a shoelace stitching pattern of commissure stitches in accordance with one embodiment.

### DETAILED DESCRIPTION

Specific embodiments are now described with reference to the figures, wherein like reference numbers indicate identical or functionally similar elements. The terms "distal" and "proximal", when used in the following description to refer to a native vessel, native valve, or a device to be implanted into a native vessel or native valve, such as a heart valve prosthesis, are with reference to the direction of blood flow. Thus, "distal" and "distally", also referred to as the "outflow" or "outflow direction", respectively, refer to positions in a downstream direction with respect to the direction of blood flow. The terms "proximal" and "proximally", also referred to as the "inflow" or "inflow direction", respectively, refer to positions in an upstream direction with respect to the direction of blood flow.

Although the description is in the context of treatment of an aortic heart valve, embodiments may also be used where it is deemed useful in other valved intraluminal sites that are not in the heart. For example, embodiments may be applied to other heart valves or venous valves as well.

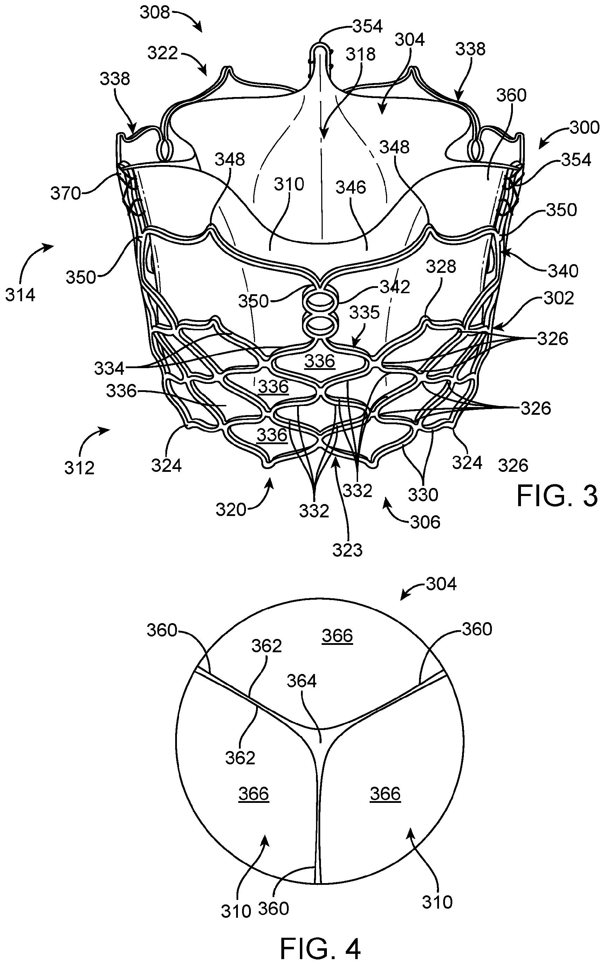

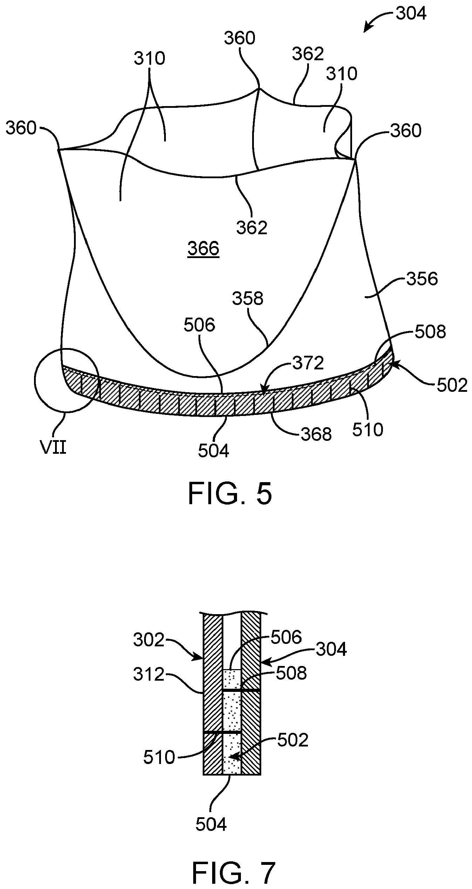

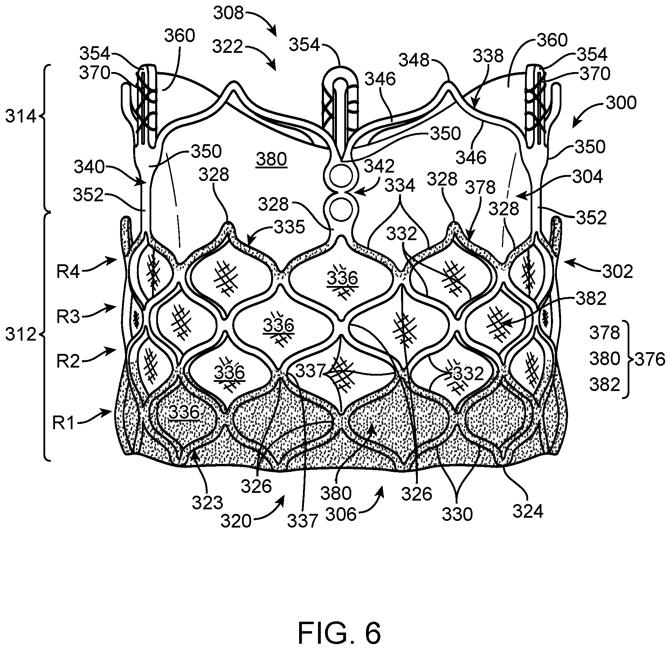

FIG. 3 is a perspective view of a transcatheter valve prosthesis 300 in an expanded configuration in accordance with one embodiment. FIG. 4 is an outflow end view of a prosthetic valve 304 of valve prosthesis 300 of FIG. 3 in accordance with one embodiment. FIG. 5 is a perspective view of prosthetic valve 304 including a reinforcement member 502 of valve prosthesis 300 of FIG. 3 in accordance with one embodiment. FIG. 6 is a side view of valve prosthesis 300 of FIG. 3 in accordance with one embodiment. FIG. 4 illustrates prosthetic valve 304 in a closed state while FIGS. 3, 5 and 6 illustrates prosthetic valve 304 in an open state.

Referring now to FIGS. 3-6 together, valve prosthesis 300 has a radially-expandable stent structure 302, sometimes called the frame or frame structure, and prosthetic valve 304. Stent structure 302 is generally tubular, and is mechanically or balloon expandable, having a crimped configuration for delivery within a vasculature and an expanded configuration for deployment within a native heart valve. When valve prosthesis 300 is deployed within the valve annulus of a native heart valve, stent structure 302 of valve prosthesis 300 is configured to be radially expanded within native valve leaflets of the patient's defective valve, to thereby retain the native valve leaflets in a permanently open state. In embodiments hereof, valve prosthesis 300 is configured for replacement of an aortic valve such that an inflow end 306, i.e., the proximal end, of valve prosthesis 300 extends into and anchors within the aortic annulus of a patient's left ventricle, while an outflow end 308, i.e., the distal end, of valve prosthesis 300 is positioned within the aortic sinuses.

Stent structure 302 of valve prosthesis 300 may be a unitary frame or scaffold that supports prosthetic valve 304 including one or more valve leaflets 310 within the interior of stent structure 302. Prosthetic valve 304 is capable of blocking flow in one direction to regulate flow there-through via valve leaflets 310 that may form a bicuspid or tricuspid replacement valve. Although prosthetic valve 304 is illustrated and discussed herein as having three valve leaflets 310, i.e., prosthetic valve 304 has a tricuspid leaflet configuration, a bicuspid leaflet configuration may be used in other embodiments. More particularly, as valve prosthesis 300 is configured for placement within a native aortic valve which typically has three leaflets, prosthetic valve 304 may include three valve leaflets 310. However, valve prosthesis 300 is not required to have the same number of leaflets as the native valve. If valve prosthesis 300 is alternatively configured for placement within a native valve having two leaflets such as the mitral valve, prosthetic valve 304 may include two or three valve leaflets 310.

Stent structure 302 is balloon-expandable. As such, stent structure 302 is made from a plastically deformable material such that when expanded by a dilatation balloon, stent structure 302 maintains its radially expanded configuration. Stent structure 302 may be formed from stainless steel such as 316L or other suitable metal, such as platinum iridium, cobalt chromium alloys such as MP35N or L605, or various types of polymers or other similar materials, including the materials coated with various surface deposits to improve clinical functionality. Stent structure 302 is configured to be rigid such that it does not deflect or move when subjected to in-vivo forces, or such that deflection or movement is minimized when subjected to in-vivo forces.

Stent structure 302 includes an inflow portion 312 and an outflow portion 314. Stent structure 302 is a tubular component defining a central lumen or passageway 318, and has an inflow end 320 and an outflow end 322. When expanded, a diameter of inflow end 320 of stent structure 302 is substantially the same as a diameter of outflow end 322 of stent structure 302 in one embodiment.

Inflow portion 312 extends distally from inflow end 320 of stent structure 302. Inflow portion 312 includes inflow crowns 324, central crowns 326, outflow crowns 328, inflow struts 330, central struts 332, and outflow struts 334. Inflow portion 312 further includes an inflow crown ring 323 defined by inflow crowns 324, inflow struts 330, and central crowns 326. Inflow portion 312 further includes an outflow crown ring 335 defined by outflow crowns 328, outflow struts 334, and central crowns 326. Inflow portion 312 generally extends between inflow crown ring 323 and outflow crown ring 335.

In between inflow crown ring 323 and outflow crown ring 335, inflow portion cells 336, sometimes called side openings, are formed in rows, and more particularly, in four rows R1, R2, R3, and R4. Each row R1, R2, R3, R4 includes 12 inflow portion cells 336. Inflow portion cells 336 of the proximal most row R1 are defined by inflow crowns 324, inflow struts 330, central crowns 326, and central struts 332. Inflow portion cells 336 of the next most proximal rows R2, R3 are defined by central crowns 326 and central struts 332. Inflow portion cells 336 of the distal most row R4 are defined by outflow crowns 328, outflow struts 334, central crowns 326, and central struts 332. Generally, inflow portion cells 336 are diamond-shaped openings having the same or identical shaped, e.g., are sometimes called symmetric.

Generally, a crown is defined where two struts connect and a node is defined as a region where two crowns connect. Accordingly, inflow crowns 324 are defined where inflow struts 330 connect. Outflow crowns 328 are defined where outflow struts 334 connect. Central crowns 326 are defined where inflow struts 330 connect, where outflow struts 334 connect, and where central struts 332 connect. Central nodes 337 are defined where central crowns 326 connect.

Outflow portion 314 is formed proximate to outflow end 322 of stent structure 302 and between outflow end 322 and inflow portion 312. Outflow portion 314 includes an outflow portion crown ring 338, commissure posts 340, and non-commissure posts 342. Outflow portion 314 can be configured in a shape that forms a central lumen or passageway.

Outflow portion crown ring 338 includes outflow portion struts 346, superior crowns 348, and inferior crowns 350. Each outflow portion struts 346 is connected at one end to an adjacent outflow crown strut 346 at a superior crown 348 and on the opposite end to an adjacent outflow crown strut 346 at an inferior crown 350. Inferior crowns 350 are connected to either a commissure post 340 or a non-commissure post 342. More particularly, every other inferior crown 350 is connected to a commissure post 340 and every other inferior crown 350 is connected to a non-commissure post 342 in an alternating repeating arrangement.

Non-commissure posts 342, sometimes called axial frame members 342, extend longitudinally between and connect inferior crowns 350 of outflow portion crown ring 338 and outflow crowns 328 of inflow portion 312. In accordance with this embodiment, non-commissure posts 342 are shaped as a figure eight and can be used as markers for depth of implant as well as clocking of valve prosthesis 300. As used herein, longitudinally is in a direction parallel with the longitudinal axis, radially is perpendicular and in a radial direction from the longitudinal axis, and circumferentially is in a plane perpendicular to the longitudinal axis and in a direction along the circumference of valve prosthesis 300.

Commissure posts 340 extend longitudinally and include axial frame members 352 and trident posts 354. Axial frame members 352 extend longitudinally between and connect inferior crowns 350 of outflow portion crown ring 338 and outflow crowns 328 of inflow portion 312. Trident posts 354 extend in a cantilever fashion and in the outflow or distal direction from inferior crowns 350 of outflow portion crown ring 338. Trident posts 354 are solid linear members in one embodiment although have a central longitudinal slot in another embodiment. Axial frame members 352 and trident posts 354 are parallel with one another and are segments of commissure posts 340, which are linear members.

Prosthetic valve 304 is a one piece 3D molded structure in accordance with this embodiment. Illustratively, a single cylindrical piece is molded to form prosthetic valve 304. Accordingly, although various structures of prosthetic valve 304 are discussed below, prosthetic value 304 is integral, i.e., formed from a single piece and not a plurality of separate pieces connected together.

Prosthetic valve 304 may be made of pericardial material; however, may instead be made of another material. Natural tissue for prosthetic valve 304 may be obtained from, for example, heart valves, aortic roots, aortic walls, aortic leaflets, pericardial tissue, such as pericardial patches, bypass grafts, blood vessels, intestinal submucosal tissue, umbilical tissue and the like from humans or animals. Synthetic materials suitable for use as prosthetic valve 304 include DACRON polyester commercially, other cloth materials, nylon blends, polymeric materials, and vacuum deposition nitinol fabricated materials. One polymeric material from which prosthetic valve 304 can be made is an ultra-high molecular weight polyethylene material. With certain materials, it may be desirable to coat one or both sides of prosthetic valve 304 with a material that will prevent or minimize overgrowth. It is further desirable that the material is durable and not subject to stretching, deforming, or fatigue.

Prosthetic valve 304 includes valve leaflets 310 and a valve inflow cylinder 356 proximal of valve leaflets 310. For example, valve inflow cylinder 356 is the remaining unmolded portion of the cylindrical material used to form prosthetic valve 304 and valve leaflets 310 are the molded portion.

Valve leaflets 310 are defined by cusps 358, commissures 360, and free edges 362. Adjoining pairs of valve leaflets 310 are attached to one another at their lateral ends to form commissures 360, with free edges 362 of valve leaflets 310 forming coaptation edges that meet in an area of coaptation 364. The region within cusps 358, commissures 360, and free edges 362 are referred to as a belly 366 of valve leaflets 310.

Valve inflow cylinder 356 has a cylindrical inflow end 368, sometimes called a nadir 368. Valve inflow cylinder 356 extends in the outflow direction from inflow end 368 as a cylinder to cusps 358, which form the outflow end of valve inflow cylinder 356. Valve inflow cylinder 356 joins valve leaflets 310 at cusps 358.

Prosthetic valve 304 is disposed within and secured to at least trident posts 354 of commissure posts 340 of stent structure 302. More particularly, commissures 360 are attached to trident posts 354, e.g., with commissure stitching 370. In addition, prosthetic valve 304 may also be disposed within and secured to inflow portion 312 of stent structure 302 as discussed in more detail below.

Valve prosthesis 300 further includes a skirt 376, which encloses or lines a portion of stent structure 302. Skirt 376 is not illustrated in FIG. 3 for clarity of visualization of stent structure 302.

Skirt 376 may enclose or line stent structure 302. Skirt 376 may be a natural or biological material such as pericardium or another membranous tissue such as intestinal submucosa. Alternatively, skirt 376 may be a low-porosity woven fabric, such as polyester, Dacron fabric, or PTFE. In one embodiment, skirt 376 may be a knit or woven polyester, such as a polyester or PTFE knit, which can be utilized when it is desired to provide a medium for tissue ingrowth and the ability for the fabric to stretch to conform to a curved surface. Polyester velour fabrics may alternatively be used, such as when it is desired to provide a medium for tissue ingrowth on one side and a smooth surface on the other side.

In accordance with this embodiment, skirt 376 includes an outflow end outer skirt 378, an inflow end outer skirt 380, and an inner skirt 382. Outflow end outer skirt 378 is located on the outer surface of outflow crowns 328 and outflow struts 334 of inflow portion 312. Inflow end outer skirt 380 is located on the outer surface of inflow crowns 324, inflow struts 330, central crowns 326, and central struts 332. More particularly, inflow end outer skirt 380 covers first row R1 of inflow portion cells 336. Inner skirt 382 is located on the inner surface of inflow portion 312 and extends between inflow crown ring 323 and outflow crown ring 335.

FIG. 7 is an enlarged cross-sectional view of a region VII of prosthetic valve 304 including reinforcement member 502 of FIG. 5 including stent structure 302 of valve prosthesis 300 of FIG. 3 in accordance with one embodiment. Referring now to FIGS. 3-7 together, to provide reinforcement and strength to prosthetic valve 304, e.g., during loading, reinforcement member 502 is attached to prosthetic valve 304.

In accordance with this embodiment, reinforcement member 502 is a strip of material extending around the entire outer circumference of valve inflow cylinder 356 at cylindrical inflow end 368 of prosthetic valve 304. Reinforcement member 502 is attached to a margin of attachment (MOA) 372, e.g., a region directly adjacent cylindrical inflow end 368, of prosthetic valve 304. Reinforcement member 502 has a uniform longitudinal length between a circumferential inflow end 504 and a circumferential outflow end 506 of reinforcement member 502. Reinforcement member 502 is formed of any of the materials listed above for prosthetic valve 304 including tissue, fabric, or polymer.

Reinforcement member 502 is attached to valve inflow cylinder with reinforcement member stitching 508. In one embodiment, reinforcement member stitching 508 is a single row of stitching, e.g., suture, extending around the circumference of prosthetic valve 304 although is two or more rows of stitching in other embodiments. Reinforcement member stitching 508 is adjacent outflow end 506 of reinforcement member 502 in accordance with this embodiment, although can be positioned at other location of reinforcement member 502 in other embodiments.

Further, reinforcement member 502 is directly attached to stent structure 302 at inflow portion 312 with stent structure stitching 510. In accordance with one embodiment, inner skirt 382 is removed, e.g., at least from the region occupied by reinforcement member 502, allowing direct attachment of reinforcement member 502 to stent structure 302. As illustrated in detail in FIG. 7, reinforcement member stitching 508 extends through and attaches reinforcement member 502 to prosthetic valve 304 whereas stent structure stitching 510 extends through and attaches reinforcement member 502 and stent structure 302.

Reinforcement member 502 is additional material outside the leaflet belly region, i.e., outside of bellies 366, that gets attached directly to stent structure 302 to provide reinforcement and durability of prosthetic valve 304, e.g., during the loading phase.

Delivery of valve prosthesis 300 may be accomplished via a percutaneous transfemoral approach or a transapical approach directly through the apex of the heart via a thoracotomy, or may be positioned within the desired area of the heart via different delivery methods known in the art for accessing heart valves. During delivery, valve prosthesis 300 remains compressed until it reaches a target diseased native heart valve, at which time a balloon of a delivery system is inflated in order to radially expand valve prosthesis 300 in situ. Valve prosthesis 300 is configured to be expanded within the native valve leaflets of the patient's defective valve, to thereby retain the native valve leaflets in a permanently open state. The delivery system is then removed and transcatheter valve prosthesis 300 remains deployed within the native target heart valve.

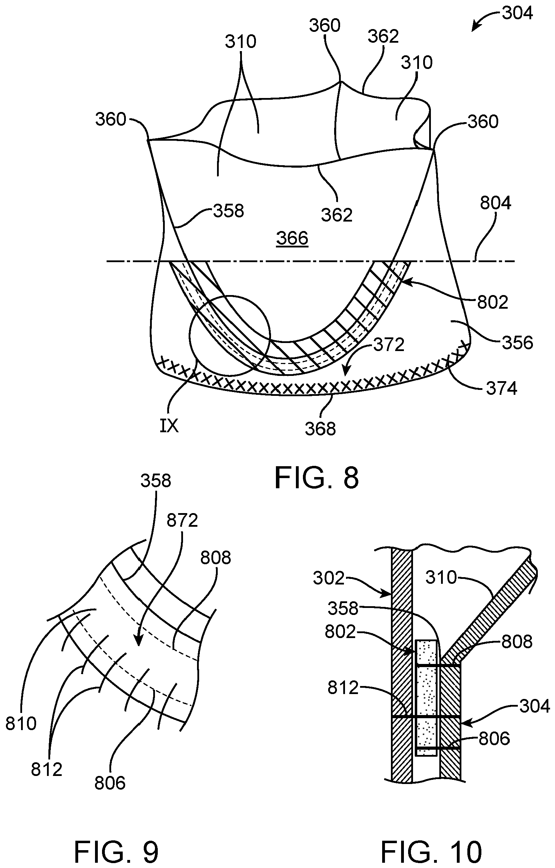

FIG. 8 is a perspective view of prosthetic valve 304 including reinforcement members 802 of valve prosthesis 300 of FIG. 3 in accordance with one embodiment. FIG. 9 is an enlarged plan view of a region IX of prosthetic valve 304 and reinforcement member 802 of FIG. 8 in accordance with one embodiment. FIG. 10 is an enlarged cross-sectional view of the region IX of prosthetic valve 304 including reinforcement member 802 of FIG. 8 including stent structure 302 of valve prosthesis 300 of FIG. 3 in accordance with one embodiment.

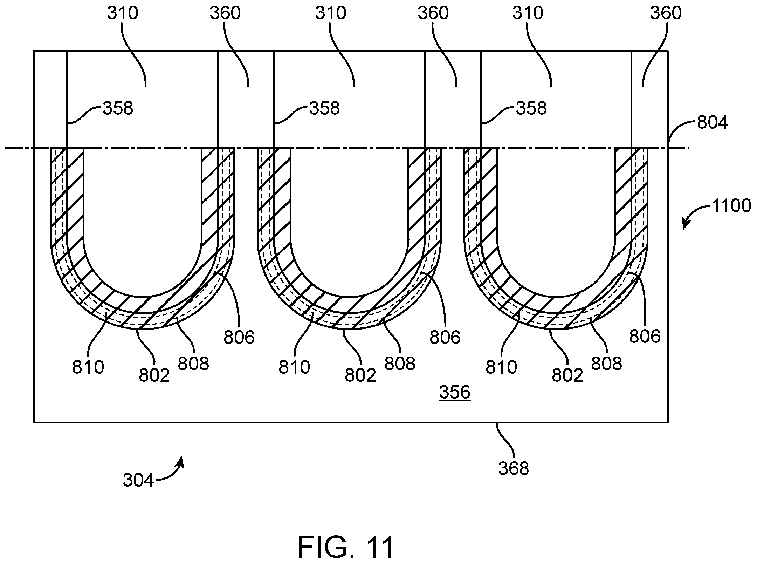

FIG. 11 is a laid flat plan view of a tissue coupon 1100 illustrating prosthetic valve 304 including reinforcement members 802 of FIGS. 8-10 in accordance with one embodiment. Tissue coupon 1100 of FIG. 11 corresponds to prosthetic valve 304 of FIG. 8 cut longitudinally at a commissure 360. It is to be understood that tissue coupon 1100 is set forth in FIG. 11 to facilitate understanding of the structure of prosthetic valve 304 and reinforcement member 802 but that prosthetic valve 304 is fabricated from a cylindrical piece of material and tissue coupon 1100 would not exist during actual fabrication.

Referring now to FIGS. 8-11 together, to provide reinforcement of prosthetic valve 304, reinforcement members 802 are attached to prosthetic valve 304. In accordance with this embodiment, reinforcement members 802 are U-shaped strips of material attached to valve inflow cylinder 356 adjacent cusps 358 of prosthetic valve 304. Reinforcement members 802 are formed of any of the materials listed above for prosthetic valve 304 including tissue, fabric, or polymer.

Reinforcement member 802 are attached proximally, sometimes called inferior, to an inferior coaptive junction (ICJ) 804 of prosthetic valve 304. Inferior coaptive junction 804 is the longitudinal position where valve leaflets 310 separate from one another proximal to being joined at commissures 360.

Generally, one reinforcement member 802 is provided adjacent each cusp 358 of each valve leaflet 310. As there are three valve leaflets 310, there are three reinforcement members 802 in this embodiment. However, there are more or less than three reinforcement members 802 in other embodiments depending upon the number of valve leaflets. In the following description, a single reinforcement member 802 and associated attachment, e.g., stitching, is discussed. However, it is to be understood that the discussion is equally applicable to all the reinforcement members 802.

In accordance with this embodiment, reinforcement member 802 is attached to prosthetic valve 304 by reinforcement stitchings 806, 808, sometimes called first and second reinforcement stitchings 806, 808. Reinforcement stitchings 806, 808 extend in a U-shape proximally following the U-shape of the respective cusp 358. Reinforcement stitchings 806, 808 extend through and attach reinforcement member 802 and valve inflow cylinder 356 of prosthetic valve 304. Reinforcement member 802 extends distally from valve inflow cylinder 356 to overlap cusp 358, without direct attachment to valve leaflet 310.

Reinforcement stitching 806 is spaced apart from reinforcement stitching 808. In this particular embodiment, reinforcement stitching 806 is located proximally from reinforcement stitching 808. In one embodiment, a space 810 between reinforcement stitchings 806, 808 forms a margin of attachment 872, which is strengthened by reinforcement stitchings 806, 808. Margin of attachment 872 is a region of valve inflow cylinder 356 and reinforcement member 802 just proximal to cusp 358.

Margin of attachment 872 is directly attached to stent structure 302, e.g., at inflow portion 312, with stent structure stitching 812. In accordance with one embodiment, inner skirt 382 is removed, e.g., at least from the region occupied by reinforcement member 802, allowing direct attachment of reinforcement member 802 to stent structure 302. As illustrated in detail in FIG. 10, reinforcement stitchings 806, 808 extends through and attaches reinforcement member 802 to prosthetic valve 304 whereas stent structure stitching 812 extends through and attaches both reinforcement member 802 and prosthetic valve 304 to stent structure 302.

Reinforcement member 802 is located between prosthetic valve 304 and stent structure 302. Reinforcement member 802 is located between cusp 358 and stent structure 302 preventing valve leaflet 310 from hitting or otherwise contacting stent structure 302. At the same time, reinforcement member 802 is located at margin of attachment 872 where stent structure stitching 812 is attached to provide more strength as compared to prosthetic valve 304 alone.

Although both reinforcement stitching 806 and reinforcement stitching 808 are illustrated and discussed, in another embodiment, only reinforcement stitching 806 and reinforcement stitching 808 is provided. In yet another embodiment, more than two reinforcement stitchings similar to reinforcement stitching 806 and reinforcement stitching 808 are provided. In yet another embodiment, one or more of reinforcement stitching 806 and reinforcement stitching 808 are provided and reinforcement member 802 is not provided or used.

Further, paying particular attention now to FIG. 8, a proximal margin of attachment (MOA) 372 of valve inflow cylinder 356, e.g., a region directly adjacent cylindrical inflow end 368, is attached to inflow portion 312. Proximal margin of attachment 372 of valve inflow cylinder 356 is sutured or otherwise securely and sealingly attached to inflow portion 312, e.g., with MOA stitching 374. However, in one embodiment, attachment of proximal margin of attachment 372 to stent structure 302 is unnecessary and so MOA stitching 374 is not provided or used.

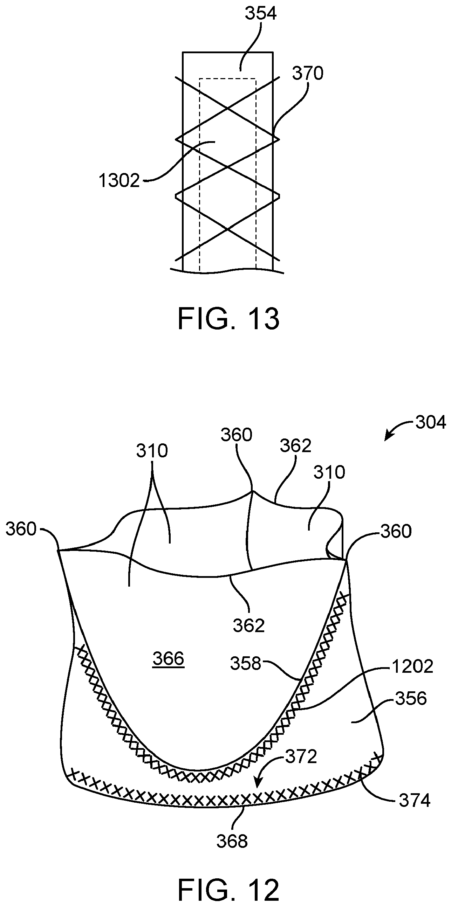

FIG. 12 is a perspective view of prosthetic valve 304 including tacking stitching 1202 of valve prosthesis 300 of FIG. 3 in accordance with one embodiment. Referring now to FIG. 12, tacking stitching 1202, sometimes called a tacking suture, is applied to prosthetic valve 304 and to inner skirt 382. As discussed above, inner skirt 382 is attached to inflow portion 312 of stent structure 302, e.g., with stitching (inner skirt 382 and stent structure 302 including inflow portion 312 are illustrated in FIG. 6). In this manner, prosthetic valve 304 is attached to stent structure 302 by tacking stitching 1202.

Tacking stitching 1202 provides additional tacking to avoid undesirable bulging of prosthetic valve 304, e.g., bulging inward and away from stent structure 302. In accordance with this embodiment, tacking stitching 1202 is provided on valve inflow cylinder 356 adjacent cusps 358, along belly 366 and the inferior coaptive area (ICA).

Although a particular location for providing tacking stitching 1202 is illustrated in FIG. 12 and discussed above, the number of stitches and stitching location varies in other embodiments depending upon the tacking requirement, e.g., to avoid bulging.

FIG. 13 illustrates a shoelace stitching pattern of commissure stitches 370 in accordance with one embodiment. Referring to FIG. 3, 6, 13, as an alternative to the pattern of commissure stitches 370 illustrated in FIGS. 3, 6, commissure stitches 370 are formed into a shoelace stitching pattern in FIG. 13. More particularly, commissure stitches 370 cross one another from one side of trident post 354 to the other side of trident post 354 in an alternating repeating pattern similar to the pattern of a lace of a shoe, i.e., in a shoelace stitching pattern.

Although trident post 354 is illustrated as a solid member in FIG 13, in another embodiment, trident post 354 includes a slot 1302 (indicated in the dashed lines) through which a commissure 360 of prosthetic valve 304 is passed and then stitched using commissure stitches 370.

It should be understood that various aspects disclosed herein may be combined in different combinations than the combinations specifically presented in the description and accompanying drawings. It should also be understood that, depending on the example, certain acts or events of any of the processes or methods described herein may be performed in a different sequence, may be added, merged, or left out altogether (e.g., all described acts or events may not be necessary to carry out the techniques). In addition, while certain aspects of this disclosure are described as being performed by a single module or unit for purposes of clarity, it should be understood that the techniques of this disclosure may be performed by a combination of units or modules associated with, for example, a medical device.

Further disclosed herein is the subject-matter of the following clauses:
1. A valve prosthesis comprising:
   a one piece molded prosthetic valve; and
   a reinforcement member attached to the prosthetic valve directly adjacent a cylindrical inflow end of the prosthetic valve.
2. The valve prosthesis of Clause 1 wherein the valve prosthesis comprises valve leaflets and a valve inflow cylinder proximal of the valve leaflets, the reinforcement member being attached to the valve inflow cylinder.
3. The valve prosthesis of Clause 2 wherein the reinforcement member extends around an entire outer circumference of the valve inflow cylinder.
4. The valve prosthesis of Clause 1 or of any of the preceding Clauses, wherein the reinforcement member has a uniform longitudinal length between a circumferential inflow end of the reinforcement member and a circumferential outflow end of the reinforcement member.
5. The valve prosthesis of Clause 4 further comprising reinforcement member stitching attaching the reinforcement member to the prosthetic valve, the reinforcement member stitching extending around an entire circumference of the prosthetic valve adjacent the outflow end of the reinforcement member.
6. The valve prosthesis of Clause 5 further comprising:
   a stent structure comprising an inflow portion; and
   stent structure stitching attaching the reinforcement member to the inflow portion.
7. A valve prosthesis comprising:
   a one piece molded prosthetic valve comprising:
      a valve leaflet comprising a cusp; and
      a valve inflow cylinder proximal of the valve leaflet; and
   a reinforcement member attached to the valve inflow cylinder adjacent the cusp.
8. The valve prosthesis of Clause 7 wherein the reinforcement member is U-shaped.
9. The valve prosthesis of Clause 7 or of any of Clauses 7-8, further comprising a first reinforcement stitching attaching the reinforcement member to the valve inflow cylinder.
10. The valve prosthesis of Clause 9 further comprising a second reinforcement stitching attaching the reinforcement member to the valve inflow cylinder.
11. The valve prosthesis of Clause 10 wherein a space between the first reinforcement stitching and the second reinforcement stitching defines a margin of attachment proximal to the cusp.
12. The valve prosthesis of Clause 11 further comprising:
   a stent structure; and
   stent structure stitching attaching the margin of attachment to the stent structure.
13. The valve prosthesis of Clause 12 wherein the reinforcement member prevents direct contact between the valve leaflet and the stent structure.
14. The valve prosthesis of Clause 7 or of any of Clauses 7-13, wherein the reinforcement member extends distally from the valve inflow cylinder to overlap the cusp.
15. A valve prosthesis comprising:
   a one piece molded prosthetic valve comprising:
      valve leaflets; and
      a valve inflow cylinder proximal of the valve leaflets;
   a stent structure;
   an inner skirt attached to the stent structure; and
   tacking stitching tacking the valve inflow cylinder to the inner skirt.
16. The valve prosthesis of Clause 15 wherein the tacking stitching prevents inward bulging of the prosthetic valve.
17. The valve prosthesis of Clause 15 or of any of Clauses 15-16, wherein the valve leaflets are defined by cusps, the tacking stitching being provided on the valve inflow cylinder adjacent the cusps.

## Claims

1. A valve prosthesis comprising:
a one piece molded prosthetic valve; and
a reinforcement member attached to the prosthetic valve directly adjacent a cylindrical inflow end of the prosthetic valve.

2. The valve prosthesis of Claim 1 wherein the valve prosthesis comprises valve leaflets and a valve inflow cylinder proximal of the valve leaflets, the reinforcement member being attached to the valve inflow cylinder.

3. The valve prosthesis of Claim 2 wherein the reinforcement member extends around an entire outer circumference of the valve inflow cylinder.

4. The valve prosthesis of any of the preceding Claims, wherein the reinforcement member has a uniform longitudinal length between a circumferential inflow end of the reinforcement member and a circumferential outflow end of the reinforcement member.

5. The valve prosthesis of Claim 4 further comprising reinforcement member stitching attaching the reinforcement member to the prosthetic valve, the reinforcement member stitching extending around an entire circumference of the prosthetic valve adjacent the outflow end of the reinforcement member.

6. The valve prosthesis of Claim 5 further comprising:
a stent structure comprising an inflow portion; and
stent structure stitching attaching the reinforcement member to the inflow portion.

7. A valve prosthesis comprising:
a one piece molded prosthetic valve comprising:
a valve leaflet comprising a cusp; and
a valve inflow cylinder proximal of the valve leaflet; and
a reinforcement member attached to the valve inflow cylinder adjacent the cusp.

8. The valve prosthesis of Claim 7 wherein the reinforcement member is U-shaped.

9. The valve prosthesis of any of Claims 7-8 further comprising a first reinforcement stitching attaching the reinforcement member to the valve inflow cylinder.

10. The valve prosthesis of Claim 9 further comprising a second reinforcement stitching attaching the reinforcement member to the valve inflow cylinder.

11. The valve prosthesis of Claim 10 wherein a space between the first reinforcement stitching and the second reinforcement stitching defines a margin of attachment proximal to the cusp.

12. The valve prosthesis of Claim 11 further comprising:
a stent structure; and
stent structure stitching attaching the margin of attachment to the stent structure, wherein typically the reinforcement member prevents direct contact between the valve leaflet and the stent structure.

13. The valve prosthesis of any of Claims 7-12 wherein the reinforcement member extends distally from the valve inflow cylinder to overlap the cusp.

14. A valve prosthesis comprising:
a one piece molded prosthetic valve comprising:
valve leaflets; and
a valve inflow cylinder proximal of the valve leaflets;
a stent structure;
an inner skirt attached to the stent structure; and
tacking stitching tacking the valve inflow cylinder to the inner skirt.

15. The valve prosthesis of Claim 14 wherein the tacking stitching prevents inward bulging of the prosthetic valve, and/or wherein the valve leaflets are defined by cusps, the tacking stitching being provided on the valve inflow cylinder adjacent the cusps.
